# EUROPEAN PATENT SPECIFICATION

(11) **EP 0 518 838 B1**
(45) Date of publication and mention of the grant of the patent: **11.10.1995**
(21) Application number: 92850137.8
(22) Date of filing: 10.06.1992
(51) Int. Cl.: A61F 2/06

(54) **Transluminal implantation device**
Transluminale Implantationsvorrichtung
Dispositif d'implantation transluminale

(30) Priority: 14.06.1991 SE 9101841
(43) Date of publication of application: 16.12.1992
(73) Proprietor: AMS MEDINVENT S.A., CH-1003 Lausanne (CH)
(72) Inventor: Bachmann, Michel, CH-1032 Romanel (CH)
(74) Representative: Burman, Tore

(56) References cited:
- EP-A- 0 364 420
- EP-A- 0 408 245
- FR-A- 2 525 896
- FR-A- 2 573 986

## Description

The present invention relates to a device for transluminal implantation of a substantially tubular, radially expansible stent, as specified in the preamble of Claim 1. Such a device is known from US-A-4655721 (corresponding to FR-A-2525896).

Devices for transluminal implantation of expanding stents or prostheses are previously known. Thus, US patent 4,732,152 describes a device enabling transluminal implantation of self-expanding stents. The device described in said US patent shows excellent performance in regard to enabling implantation of prostheses or stents in for example blood vessels or other ducts in living animal bodies. Said US patent exemplifies operation with prostheses or stents of the type disclosed in US patent 4,655,771. This particular type of prostheses for use in transluminal implantation comprises a flexible tubular body composed of a braided arrangement of flexible thread elements involving free ends at the axial extremes of the prostheses. Although the present invention is not limited to transluminal implantation of the type of prostheses or stents disclosed in US patent 4,655,771 the present invention will be illustrated with special reference to such stents. In practical operation it has been found that handling such stents in connection with transluminal implantation thereof involves practical problems related to particularly the rear end of the stent and its association with the implantation device. Thus, it has been found that radial observation openings may be needed in the concentric tubes involved in connection with the implantation procedure in order to properly locate the stent before its release at the site of implantation. Such observation openings result in practical complications due to interaction with the rear end of the stent which may result in operational problems and even failure to provide for proper implantation.

The present invention has for its main object to solve the problems associated with implantation devices capable of providing for insertion and release of prostheses or stents of different types, for example of the type disclosed in US patent 4,655,771.

Another object of the invention is to provide for a device that provides for safety operation in the implantation procedure and enabling proper positioning of expanding stents in connection with their implantation.

For these and other objects which will be better understood by the following disclosure the invention provides a device for transluminal implantation of a substantially tubular, radially expansible stent, as defined in Claim 1.

In the instant disclosure the expressions "distal" and "proximal" refer to the stent end and the handle end, respectively, of the associated construction detail or element.

In a preferred embodiment of the device of the invention said central tube or rod is provided at the distal end thereof with at least two radially extending members engaging the stent from the inside to facilitate its release. The number of said members is preferably at least three, and the members are preferably substantially evenly circumferentially distributed around said distal end.

It is preferred that the outer tube is provided with radial openings in the area thereof comprising the location of the stent so as to enable inspection of the position of the stent during its implantation. As an alternative said outer tube can be made of a transparent material to enable such inspection.

In the device according to the invention it is preferred that the axial displacement means enabling axial relative movement between the central tube or rod and the outer tube includes safety catches defining the extreme axial positions of the outer tube. The purpose of the safety catches is to avoid untimely deployment of the stent during the insertion procedure or complete release before the correct position has been reached.

In a particularly preferred embodiment of the device according to the invention the central tube is capable of accommodating viewing means, such as an endoscope or telescope, positioned inside said tube and axially displaceable therein.

The axial displacement means may be provided with a fluid inlet for flushing or for the injection of contrast media. Thus, such fluid inlet can be used for introducing flushing liquid to remove contaminants from the interior of the instrument or for introducing a contrast medium to enable X-ray inspection to facilitate positioning of the instrument during the implantation procedure.

The invention also covers an apparatus for the implantation of an expansible stent comprising an implantation device as outlined above in combination with such stent positioned in the annular space in a contracted state. Positioned in this manner the proximal or rear end of the stent is located within the undercut groove and is thus protected from damages or dislocation of its constructional elements. It is preferred that the stent is of the self-expanding type and especially preferred are stents of the type described in US patent 4,655,771. In accordance with the disclosure of said US patent the stent is in brief constituted by a flexible tubular body which has a diameter that is variable by axial movement of the ends of the body relative to each other and which is composed of several individual rigid but flexible thread elements each of which extends in helix configuration with the centre line of the body as a common axis, a number of elements having same direction of winding but being axially displaced relative to each other crossing a number of elements also axially displaced relative to each other but having the opposite direction of winding.

The present invention will now be described by a non-limiting example with reference to the appended drawings, wherein:
Fig. 1 is a diagrammatic side view of a preferred embodiment of the device of the invention;
Fig. 2 shows a detail of the device of Fig. 1;
Fig. 3 shows another detail in an exploded view of the instrument of Fig. 1;
Fig. 4 is a detail view in enlargement of the distal end of the detail of Fig. 3;
Fig. 5 is an enlarged detail view of the distal end of the instrument including a stent under release; and
Fig. 6 is an exploded side view of the device of Fig. 1 showing more in detail its different parts.

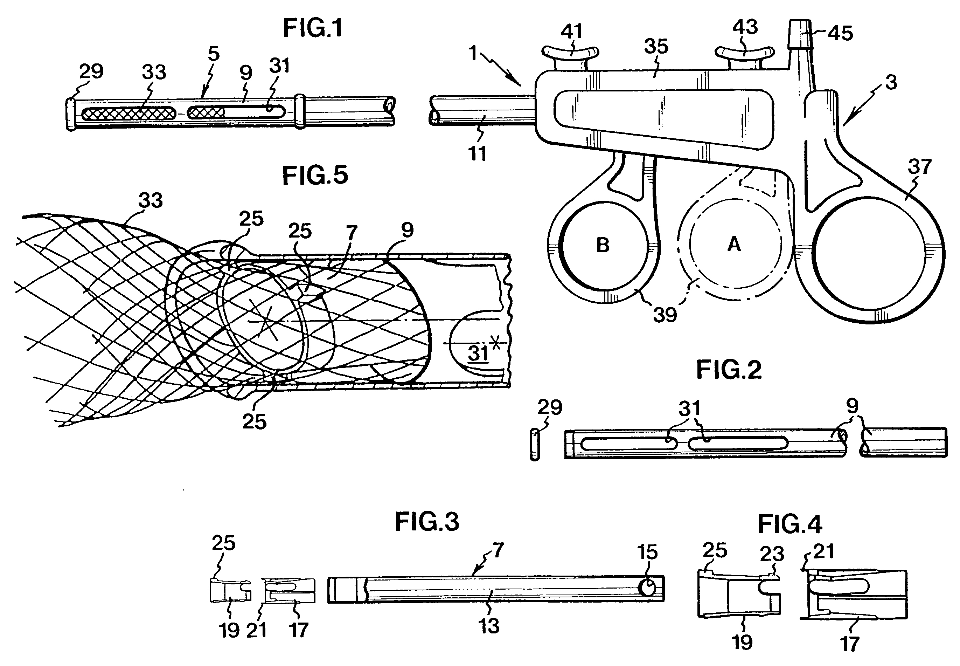

The device shown in Fig. 1 is generally designated 1 and the device comprises in principal at the proximal end thereof operating means generally designated 3 and at the distal end thereof implantation means generally designated 5. The implantation means 5 comprise a central tube generally designated 7 surrounded by an outer tube 9, said outer tube 9 at the rear or proximal end thereof being surrounded by a stabilizing exterior tube 11 attached to operating means 3.

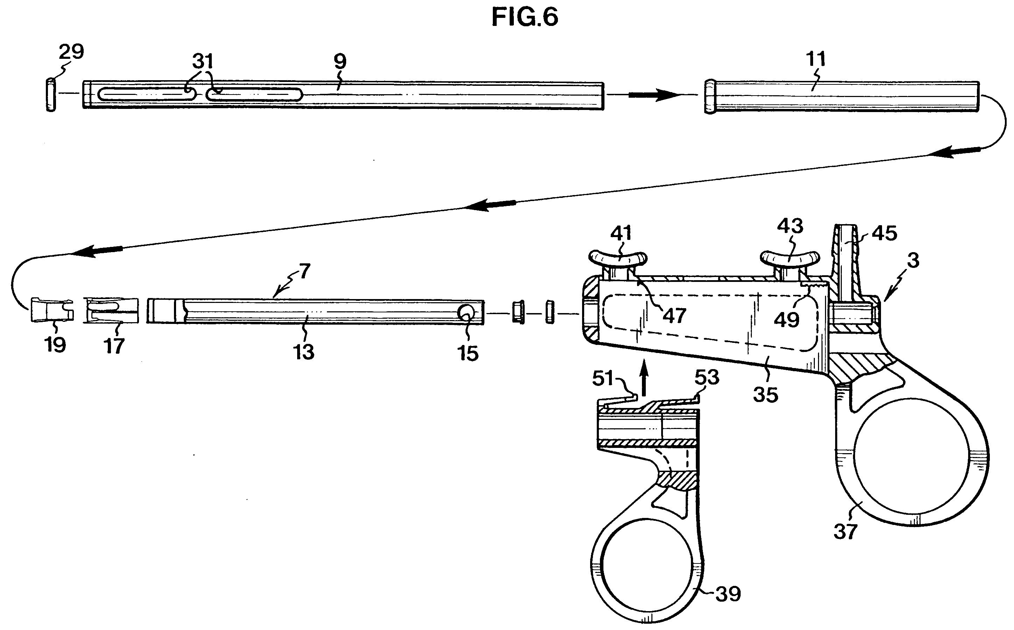

Central tube 7 is shown more in detail by exploded view in Figs. 3 and 4. Central tube 7 contains a main part 13 provided with one opening 15. Furthermore, it contains a middle part 17, the rear end of which can be inserted into and attached to the main part 13. Finally, central tube 7 includes a front end part 19 having a rear end 23 of reduced diameter and having a tapered shape for insertion into and fixation to the front end of the middle part 17. The front end part 19 of the central tube 7 is provided at its distal end with hooks or extensions 25 for a purpose to be further explained below. In the embodiment shown the number of hooks 25 is three as is evident from Fig. 5. The hooks are evenly distributed around the periphery of the front end part 19.

The middle part 17 of central tube is at its distal end provided with a forwardly directed circumferential flange 21 which when the front end part 19 is inserted into the front end of middle part 17 forms an undercut groove or recess that can accommodate the rear end of stent 33 in the position shown in Fig. 1, i.e. with the stent in a contracted state.

The outer tube 9 is shown by an exploded view in Fig. 2. At the distal end thereof it is provided with a rounded collar 29 to facilitate insertion during implantation. Furthermore, it is provided with axially extending elongate radial openings 31 for a purpose that is explained above. At the rear end thereof outer tube 9 is surrounded by a stabilizing exterior tube 11 which is attached to the operating means 3, whereas the outer tube 9 is axially displaceable within exterior tube 11.

In regard to the design of the rear end operating means 3 generally designated 3 reference is now made to Figs. 1 and 6. Fig. 1 is an assembly drawing of the device, whereas Fig. 6 shows an exploded view of the device. Operating means 3 comprise a main body 35 and integral therewith a rear loop handle 37. Said means further include a moveable second loop handle 39 which is displaceable between extreme positions within the main body 35 indicated by dashed lines and full lines in Fig. 1 illustrating retracted position and forward position, respectively. The outer tube 9 is permanently attached to the front end of the moveable second loop handle 39 and is thus axially moveable forward and backward between the extreme positions by moving loop handle 39. Operating means 3 include a front safety catch release member 41 and a rear front safety catch release member 43 corresponding to the two extreme positions indicated in Fig. 1 by positions B (dashed lines) and A (full lines), respectively. Release members 41,43 cooperate with spring elements 51 and 53, respectively, provided on the upper side of the second loop handle 39 as shown in Fig. 6. These springing elements 51,53 cooperate with safety catches 47,49 provided on the inside of main body 35. By pressing release members 41,43 springing elements 51 and 53, respectively, can be released from their engagement with the respective safety catches 47,49 so that the second loop handle 39 can be axially displaced together with the associated outer tube 9.

Finally, the main body 35 at the rear end thereof is provided with a fluid inlet 45 allowing introduction of a fluid into the instrument, such as a liquid for flushing of a contrast medium for X-ray inspection. Such fluid entering fluid inlet 45 passes through opening 15 into and through the central tube 7 for the removal of contaminants at the location of stent 33 or for providing contrast for X-ray inspection.

The function of the implantation device 1 now described is briefly the following.

Assuming introduction of a stent into a patient a stent 33 of suitable dimensions is introduced into the instrument by inserting the proximal end thereof into the annular groove formed by flange 21 of the middle part 17 of central tube 7. This procedure is performed with the second loop handle 39 in A-position as shown by dashed lines in Fig. 1. By moving the second loop handle 39 forwardly to position B outer tube 9 is moved into the position shown in Fig. 1. At this moment the safety catch involving members 47 and 51 has been activated keeping the second loop handle 39 securely in position B. Bringing stent 33 into the outer tube 9 by forward movement of the second loop handle 39 is facilitated by the engagement of hooks 25 with stent 33 from the inside thereof as seen in Fig. 5.

After introducing the distal end of device 1 into the patient at the desired location thereof as checked by for example an endoscope or telescope the forward safety catch release member 41 is activated by downward movement thereof thereby releasing the safety catch 47,51 and the second loop handle 39 with associated outer tube 9 can now be moved backward to release the stent 33 as shown in Fig. 5. During this backward movement of said handle 39 the safety catch 49,53 arrests the movement before full release of the stent. This is a safety measure and full release is obtained by pressing release member 43. When the second loop handle 39 has reached position A in Fig. 1 the stent 33 will be fully released at the desired location and the device 1 can be retracted leaving the stent 33 inside the urethra.

In the application illustrated above implantation means generally designated 5 can be made of a rigid material, whereas in other applications involving tortuous implantation paths they can be made of flexible materials enabling bending to follow the contour of the implantation path.

It is to be noted that the present invention is in no way limited to the embodiments described above. Thus, any suitable materials can be used for the different parts of the device and, furthermore, the invention is useful not only with regard to the type of stents described in US patent 4,655,771, although the device described herein is particularly useful in handling such a stent.

## Claims

1. A device (1) for transluminal implantation of a substantially tubular, radially expansible stent, comprising a central tube (7) or rod surrounded by an outer tube (9) axially displaceable relative said central tube or rod, the radial dimensions of said tubes or tube and rod being such as to form an annular space therebetween capable of accommodating said stent in an unexpanded state, and means for axial displacement (3) of said outer tube relative to said central tube or rod, said central tube or rod, at a distal end thereof, being provided with a circumferential flange (21) which forms a circumferential groove or recess within which a proximal end of said stent rests until release of the stent by rearward displacement of said outer tube (9), characterized in that said groove or recess is an undercut groove or recess formed by the combination of a section of reduced diameter provided at the distal end of said central tube (7) or rod and the said forwardly directed circumferential flange (21) arranged at the proximal end of said section of reduced diameter.

2. A device according to claim 1, wherein said central tube (7) or rod, at said distal end thereof, is provided with at least two outwardly extending members (25) engaging the stent from the inside.

3. A device according to claim 2, wherein the number of said members (25) is at least three, said members (25) being substantially evenly circumferentially distributed around said distal end.

4. A device according to any preceding claim, comprising a stabilizing tube (11) surrounding the rear part of said outer tube (9), said stabilizing tube (11) being attached at the proximal end thereof to said axial displacement means (3), and said outer tube (9) being axially displaceable within said stabilizing tube (11).

5. A device according to any of claims 1 to 4, wherein said outer tube (9) is provided with radial openings (31) to enable inspection of the position of the stent during implantation thereof.

6. A device according to any of claims 1 to 4, wherein said outer tube is made of a transparent material to enable inspection of the position of the stent during implantation thereof.

7. A device according to any preceding claim, wherein said axial displacement means (3) includes safety catches (47,49) defining extreme axial positions of said central tube or rod.

8. A device according to any preceding claim, comprising a central tube capable of accommodating viewing means, such as an endoscope or telescope positioned inside said tube and axially displaceable therein.

9. A device according to any preceding claim, wherein said axial displacement means (3) is provided with a fluid inlet (45) for flushing or contrast purposes.

10. An apparatus for implanting a substantially tubular, radially expansible stent, comprising a device (1) according to claim 1 in combination with a stent (33) positioned in said annular space in a contracted state, its proximal end being located within said undercut groove.

11. An apparatus according to claim 10, wherein said stent (33) is of the self-expanding type.

12. An apparatus according to claim 11, wherein said stent is constituted by a flexible tubular body which has a diameter what is variable by axial movement of the ends of the body relative to each other and which is composed of several individual rigid but flexible thread elements each of which extends in helix configuration with the centre line of the body as a common axis, a number of elements having same direction of winding but being axially displaced relative to each other crossing a number of elements also axially displaced relative to each other but having the opposite direction of winding.

## Patentansprüche

1. Vorichtung zur transluminalen Implantation eines im wesentlichen rohrförmigen, radial expandierbaren Offenhaltungseinsatzes, enthaltend ein zentrales Rohr (7) oder eine zentrale Stange, die von einem äußeren Rohr (9) umgeben ist, die gegenüber dem zentralen Rohr oder der zentralen Stange axial verschiebbar ist, wobei die radialen Abmessungen der Rohre oder des Rohres und der Stange so beschaffen sind, daß dazwischen ein Ringraum gebildet ist, der den Offenhaltungseinsatz in nicht expandiertem Zustand aufnehmen kann, und eine Einrichtung (3) zur axialen Verschiebung des äußeren Rohrs gegenüber dem zentralen Rohr oder der zentralen Stange, wobei das zentrale Rohr oder die zentrale Stange an ihrem distalen Ende mit einem Umfangsflansch (21) versehen ist, der eine Umfangsnut oder -ausnehmung bildet, in der ein proximales Ende des Offenhaltungseinsatzes bis zur Freigabe des Offenhaltungseinsatzes durch eine nach hinten erfolgende Verschiebung des äußeren Rohrs (9) ruht,
dadurch **gekennzeichnet,**
daß die Nut oder Ausnehmung eine hinterschnittene Nut oder Ausnehmung ist, die durch Kombination eines Abschnitts mit verringertem Durchmesser am distalen Ende des zentralen Rohrs (7) oder der zentralen Stange mit dem nach vorn gerichteten Umfangsflansch (21) am distalen Ende des Abschnitts mit verringertem Durchmesser gebildet ist.

2. Vorrichtung nach Anspruch 1, wobei das zentrale Rohr (7) oder die Stange an ihrem distalen Ende mit wenigstens zwei sich nach außen erstreckenden Gliedern (25) versehen ist, die den Offenhaltungseinsatz von innen ergreifen.

3. Vorrichtung nach Anspruch 2, wobei die Anzahl der Glieder (25) wenigstens drei beträgt, und die Glieder (25) im wesentlichen gleichmäßig in Umfangsrichtung um das distale Ende verteilt sind.

4. Vorrichtung nach einem der vorhergehenden Ansprüche, enthaltend ein Stabilisierungsrohr (11), das den hinteren Teil des äußeren Rohrs (9) ungibt, wobei das Stabilisierungsrohr (11) an seinem proximalen Ende an der Axialverschiebungseinrichtung (3) befestigt ist und das äußere Rohr (9) innerhalb des Stabilisierungsrohres axial verschiebbar ist.

5. Vorrichtung nach einem der Ansprüche 1-4, wobei das äußere Rohr (9) mit radialen Öffnungen (31) versehen ist, um eine Kontrolle der Position des Offenhaltungseinsatzes während dessen Implantation zu ermöglichen.

6. Vorrichtung nach einem der Ansprüche 1-4, wobei das äußere Rohr aus durchscheinendem Material hergestellt ist, um eine Kontrolle der Position des Offenhaltungseinsatzes während dessen Implantation zu ermöglichen.

7. Vorrichtung nach einem der vorhergehenden Ansprüche, wobei die Axialverschiebungseinrichtung (3) Sicherheitsverriegelungen (47, 49) enthält, die die axialen Endlagen des zentralen Rohrs oder der zentralen Stange festlegen.

8. Vorrichtung nach einem der vorhergehenden Ansprüche, enthaltend ein zentrales Rohr, das eine Betrachtungseinrichtung, wie ein Endoskop oder Teleskop, aufnehmen kann, die innerhalb dieses Rohrs angeordnet und darin axial verschiebbar ist.

9. Vorrichtung nach einem der vorhergehenden Ansprüche, wobei die Axialverschiebungseinrichtung (3) für Spül- oder Kontrastzwecke mit einem Fluideinlaß (45) versehen ist.

10. Gerät zur Implantation eines im wesentlichen rohrförmigen, radial expandierbaren Offenhaltungseinsatzes, enthaltend eine Vorrichtung (1) nach Anspruch 1 in Kombination mit einem Offenhaltungseinsatz (33), der in zusammengezogenem Zustand im Ringraum positioniert ist, wobei sein proximales Ende innerhalb der hinterschnittenen Nut angeordnet ist.

11. Gerät nach Anspruch 10, wobei der Offenhaltungseinsatz (33) von selbstexpandierbarer Bauart ist.

12. Gerät nach Anspruch 11, wobei der Offenhaltungseinsatz durch einen biegsamen rohrförmigen Körper gebildet ist, dessen Durchmesser durch axiales Bewegen der Enden des Körpers gegeneinander veränderlich ist und der aus mehreren einzelnen steifen, aber biegsamen Drahtelementen aufgebaut ist, von denen sich jedes mit der Mittellinie des Körpers als gemeinsamer Achse schraubenförmig erstreckt, wobei eine Anzahl von axial gegeneinander versetzten Elementen mit gleicher Wicklungsrichtung eine Anzahl von ebenfalls gegeneinander versetzten Elementen mit entgegengesetzter Wicklungsrichtung kreuzt.

## Revendications

1. Un dispositif (1) pour l'implantation transluminale d'un dilatateur radialement expansible essentiellement tubulaire comprenant un tube central (7) ou tige entouré par un tube externe (9) déplaçable axialement par rapport audit tube ou tige central, les dimensions radiales desdits tubes ou tube et tige étant telles qu'elles forment un espace annulaire entre eux capable de recevoir ledit dilatateur dans un état non dilaté et un moyen pour le déplacement axial (3) dudit tube externe par rapport audit tube ou tige central, ledit tube ou tige central, à son extrémité distale, étant pourvu d'un rebord circonférentiel (21) qui forme une gorge ou évidement circonférentiel dans lequel une extrémité proximale dudit dilatateur repose jusqu'à la libération du dilatateur par le déplacement vers l'arrière dudit tube externe (9), caractérisé en ce que ladite gorge ou évidement est une gorge ou évidement découpé formé par la combinaison d'une section de diamètre réduit prévue à l'extrémité distale dudit tube central (7) ou tige et ledit rebord circonférentiel dirigé vers l'avant (21) est disposé à l'extrémité proximale de ladite section de diamètre réduit.

2. Un dispositif selon la revendication 1, dans lequel ledit tube central (7) ou tige, à son extrémité distale est muni d'au moins deux éléments se prolongeant vers l'extérieur (25) venant au contact du dilatateur à partir de l'intérieur.

3. Un dispositif selon la revendication 2, dans lequel le nombre desdits éléments (25) est au moins trois, lesdits éléments (25) étant essentiellement répartis uniformément circonférentiellement autour de ladite extrémité distale.

4. Un dispositif selon l'une quelconque des revendications précédentes comprenant un tube de stabilisation (11) entourant la partie arrière dudit tube externe (9), ledit tube de stabilisation (11) étant fixé à son extrémité proximale audit moyen de déplacement axial (3) et ledit tube externe (9) étant déplaçable axialement dans ledit tube de stabilisation (11).

5. Un dispositif selon l'une quelconque des revendications 1 à 4, dans lequel ledit tube externe (9) est muni d'ouvertures radiales (31) pour permettre une inspection de la position du dilatateur pendant son implantation.

6. Un dispositif selon l'une quelconque des revendications 1 à 4, dans lequel ledit tube externe est réalisé en un matériau transparent pour permettre l'inspection de la position du dilatateur pendant son implantation.

7. Un dispositif selon l'une quelconque des revendications précédentes, dans lequel ledit dispositif de déplacement axial (3) comprend des cliquets de sécurité (47, 51) définissant des positions axiales extrêmes dudit tube central ou tige.

8. Un dispositif selon l'une quelconque des revendications précédentes comprenant un tube central capable de recevoir un moyen d'observation comme un endoscope ou un télescope disposé à l'intérieur dudit tube et déplaçable axialement dans celui-ci.

9. Un dispositif selon l'une quelconque des revendications précédentes, dans lequel ledit moyen de déplacement axial (3) est muni d'une entrée de fluide (45) pour le nettoyage ou à des fins de contraste.

10. Un appareil pour implanter un dilatateur radialement expansible essentiellement tubulaire comprenant un dispositif (1) selon la revendication 1 en combinaison avec un dilatateur (33) disposé dans ledit espace annulaire selon un état contracté, son extrémité proximale étant située dans ladite gorge découpée.

11. Un appareil selon la revendication 10, dans lequel ledit dilatateur (33) est du type autodilatable.

12. Un appareil selon la revendication 11, dans lequel ledit dilatateur est constitué d'un corps tubulaire souple qui a un diamètre qui est variable par mouvement axial des extrémités du corps les unes par rapport aux autres et qui est constitué de plusieurs éléments filetés individuels rigides mais souples dont chacun se prolonge selon une configuration en hélice avec la ligne centrale du corps selon un axe commun, plusieurs éléments ayant la même direction d'enroulement mais étant déplacés axialement les uns par rapport aux autres croisant plusieurs éléments également déplacés axialement les uns par rapport aux autres mais ayant la direction opposée d'enroulement.
